# DEMANDE DE BREVET EUROPEEN

(11) **EP 0 617 974 A1**
(43) Date de publication de la demande: **05.10.1994**
(21) Numéro de dépôt: 93400817.8
(22) Date de dépôt: 30.03.1993
(51) Int. Cl.: A61M 1/00

(54) **Dispositif de drainage implantable pour le traitement de l'hydrocéphalie**

(71) Demandeur: CORDIS S.A., F-91170 Viry-Châtillon (FR)
(72) Inventeur: Sierra, Rolando, F-06130 Grasse (FR)
(74) Mandataire: Bloch, Gérard

(57) **Abrégé**

La présente invention concerne un dispositif de drainage implantable pour le traitement de l'hydrocéphalie. A cet effet, il comprend une première valve de régulation de pression (1; 40,42; 52,57) en série avec une valve de régulation de débit (2; 41,44; 53,57), et une deuxième valve de régulation de pression (3; 46) en parallèle avec ladite valve de régulation de débit, la deuxième valve de régulation de pression ayant une pression différentielle d'ouverture supérieure à celle de la première.

## Description

La présente invention concerne un dispositif de drainage implantable pour le traitement de l'hydrocéphalie.

On connaît déjà divers types de valves destinées à contrôler le drainage d'un excès de fluide céphalo-rachidien contenu dans les ventricules cérébraux, vers par exemple la cavité péritonéale.

C'est ainsi que l'on a déjà proposé d'utiliser de simples clapets régulateurs de pression qui s'ouvrent au-delà d'un certain seuil de pression différentielle entre leur chambre amont et leur chambre aval, et qui empêchent ensuite cette pression différentielle de dépasser la valeur de seuil.

Ces valves présentent le sérieux inconvénient de ne pas tenir compte des différences de hauteur de colonne de liquide en fonction de la position, notamment debout ou couchée, du patient.

C'est la raison pour laquelle on a proposé un autre type de valve dans laquelle une membrane soumise à la pression différentielle entre l'amont et l'aval possède un orifice calibré coopérant avec une tige usinée traversant cet orifice. Plus la pression différentielle est importante et plus la membrane est par conséquent déformée, plus forte est la restriction au passage du fluide entre les bords de l'orifice et la tige, du fait de la section particulière donnée à cette dernière. Par conséquent ce dispositif fonctionne comme un régulateur de débit puisqu'il offre une plage de pression dans laquelle le débit reste sensiblement constant.

Un seuil inférieur de pression est obtenu en prévoyant un siège sur lequel s'appuie le bord de l'orifice calibré tant que la pression différentielle est inférieure à cette valeur de seuil. Enfin, la pression différentielle ne peut dépasser une valeur maximale provoquant une déformation de la membrane telle que la tige sort de l'orifice calibré et qu'en conséquence toute la surface de cet orifice est disponible pour l'écoulement du fluide.

Bien que donnant satisfaction une telle valve présente l'inconvénient de la difficulté de l'usinage de la tige et de la complexité de son réglage.

La présente invention vise à palier ces inconvénients en fournissant une valve présentant sensiblement les mêmes caractéristiques pression différentielle/débit que la valve précitée, mais de fabrication beaucoup plus aisée.

A cet effet, l'invention a pour objet un dispositif de drainage implantable pour le traitement de l'hydrocéphalie, caractérisé par le fait qu'il comprend une première valve de régulation de pression en série avec une valve de régulation de débit, et une deuxième valve de régulation de pression en parallèle avec ladite valve de régulation de débit, la deuxième valve de régulation de pression ayant une pression différentielle d'ouverture supérieure à celle de la première.

En conséquence on prévoit selon l'invention trois valves distinctes ayant chacune une fonction particulière et dont la combinaison permet d'obtenir la caractéristique souhaitée.

En effet, à partir d'une première valeur de pression différentielle, la première valve de régulation de pression s'ouvre et fournit par conséquent la valeur de seuil. A partir de cet instant, la valve de régulation de débit en série avec la première valve de régulation de pression assure la régulation du débit à une valeur constante quelque soit la valeur de la pression différentielle, jusqu'à une valeur maximum ou valeur de coupure à laquelle s'ouvre la deuxième valve de régulation de pression en parallèle avec la valve de régulation de débit.

Le fait de séparer les trois fonctions en trois valves différentes permet de simplifier la fabrication et le réglage du dispositif selon l'invention.

Dans un mode de réalisation avantageux de l'invention, la première valve de régulation de pression est disposée en série avec l'ensemble constitué par la valve de régulation de débit en parallèle avec la deuxième valve de régulation de pression.

Bien qu'un tel agencement ne soit pas absolument nécessaire (la deuxième valve de régulation de pression pourrait être mis en parallèle avec l'ensemble constitué par la première valve de régulation de pression en série avec la valve de régulation de débit), il est cependant préférable, notamment lorsque l'on utilise comme valve de régulation de débit une valve qui, tout en régulant le débit à une valeur donnée pour une pression différentielle inférieure à un certain seuil, se ferme une fois ce seuil dépassé.

Il est en effet alors important que cette valeur de seuil de fermeture soit au moins égale à la pression différentielle d'ouverture pour la deuxième valve de régulation de pression. Or, il est plus facile d'assurer la réalisation de cette condition lorsque la valve de régulation de débit est seule en parallèle avec la deuxième valve de régulation de pression, puisque l'on peut alors concevoir des agencements dans lesquels la fermeture de la valve de régulation de débit permet néanmoins un certain passage du liquide.

La valve de régulation de débit peut comprendre une membrane délimitant avec un boîtier une chambre amont et une chambre aval, ladite membrane de la valve de régulation de débit possédant une ouverture centrale agencée pour faire communiquer les chambres amont et aval et délimitée par un joint annulaire situé en vis à vis d'un siège de forme correspondante formé dans le boîtier du côté de la chambre aval.

On comprend que, plus la pression différentielle entre la chambre amont et la chambre aval sera forte, plus la membrane sera déformée et par conséquent plus son joint annulaire sera proche de son siège. Le passage annulaire d'écoulement de fluide entre le joint et le siège sera donc progressivement restreint mais, du fait de l'augmentation de la pression différentielle, la vitesse du fluide au niveau du passage sera simultanémént augmentée de sorte que le débit restera sensiblement constant jusqu'à ce que la pression différentielle vienne appliquer le joint contre le siège provoquant ainsi la fermeture de la valve.

Plus particulièrement le joint annulaire et/ou le siège peuvent comporter des moyens permettant une légère fuite de la valve de régulation de débit dans son état fermé, de manière à assurer un drainage des zônes de contact entre le joint et le siège empêchant aux protéines du fluide céphalo-rachidien de se retrouver pincées entre le joint et le siège et de perturber le fonctionnement du dispositif.

Dans un mode de réalisation particulier de l'invention, l'une ou moins des valves de régulation de pression comprend une membrane délimitant avec un boîtier une chambre amont et une chambre aval, ladite membrane de la valve de régulation de pression possédant une ouverture centrale agencée pour faire communiquer les chambres amont et aval et délimitée par un joint annulaire situé en vis à vis d'un siège de forme correspondante formé dans le boîtier du côté de la chambre amont, le joint étant appliqué contre le siège pour une pression différentielle entre les chambres amont et aval inférieure à la pression de régulation de ladite valve.

Dans le cas où la première valve de régulation de pression est ainsi agencée, on utilise avantageusement une seule membrane comme membrane de la valve de régulation de débit et comme membrane de la première valve de régulation de pression, un joint annulaire étant formé de chaque côté de la membrane autour de la même ouverture centrale, et les deux sièges étant en conséquence formés en vis à vis dans le boîtier, l'un du côté de la chambre amont et l'autre du côté de la chambre aval.

Une telle disposition permet une réalisation simple et compacte du dispositif selon l'invention sans pour autant en compliquer le réglage, la valeur de seuil de la première valve de régulation de pression et la valeur de fermeture de la valve de régulation de débit pouvant être, comme on le verra ci-après, réglées indépendamment l'un de l'autre.

Dans un mode de réalisation préféré de l'invention, le conduit d'amenée à la deuxième valve de régulation de pression débouche dans la chambre aval à l'intérieur de la zone délimitée par le siège de la valve de régulation de débit.

Une telle disposition assure une grande compacité au dispositif selon l'invention.

Afin de permettre un réglage aisé et indépendant des différentes valeurs de débit et de pression caractéristiques du dispositif, l'un au moins des sièges peut être formé dans une vis coopérant avec un filetage formé dans la paroi du boîtier.

En tournant cette vis, et par conséquent en la faisant plus ou moins pénétrer axialement dans le boîtier, il est possible de régler indépendamment la valeur de la pression d'ouverture de la première valve de régulation de pression et la pression de fermeture de la valve de régulation de débit.

La deuxième valve de régulation de pression peut-être une valve à fente disposée dans un perçage traversant axialement la vis subportant le siège de la valve de régulation de débit.

Dans un autre mode de réalisation de l'invention, la première valve de régulation de pression et la valve de régulation de débit sont formées d'une chambre cylindrique, dans laquelle une bille est agencée pour se déplacer entre deux sièges côniques d'extrémité, le conduit d'entrée à ladite première valve de régulation de pression débouchant au centre du premier de ces sièges côniques et le conduit de sortie de la valve de régulation de débit débouchant au centre du deuxième de ces sièges côniques, un ressort de tarage poussant ladite bille en direction du premier siège cônique, et le conduit d'amenée à la deuxième valve de régulation de pression débouchant dans ladite chambre cylindrique.

On décrira maintenant à titre d'exemple non limitatif des modes de réalisation particulier de l'invention en référence aux dessins schématiques annexés dans lesquels :
- - la figure 1: est un schéma par bloc du dispositif selon l'invention;
- - la figure 2: représente la courbe caractéristique pression/débit d'une valve de régulation de débit;
- - la figure 3: représente la courbe caractéristique pression/débit d'une valve de régulation de pression;
- - la figure 4: représente la courbe caractéristique pression/débit du dispositif selon l'invention;
- - la figure 5: est une vue en coupe axiale d'un dispositif selon un premier mode de réalisation de l'invention; et
- - la figure 6: est une vue en coupe axiale d'un dispositif selon un deuxième mode de réalisation de l'invention.

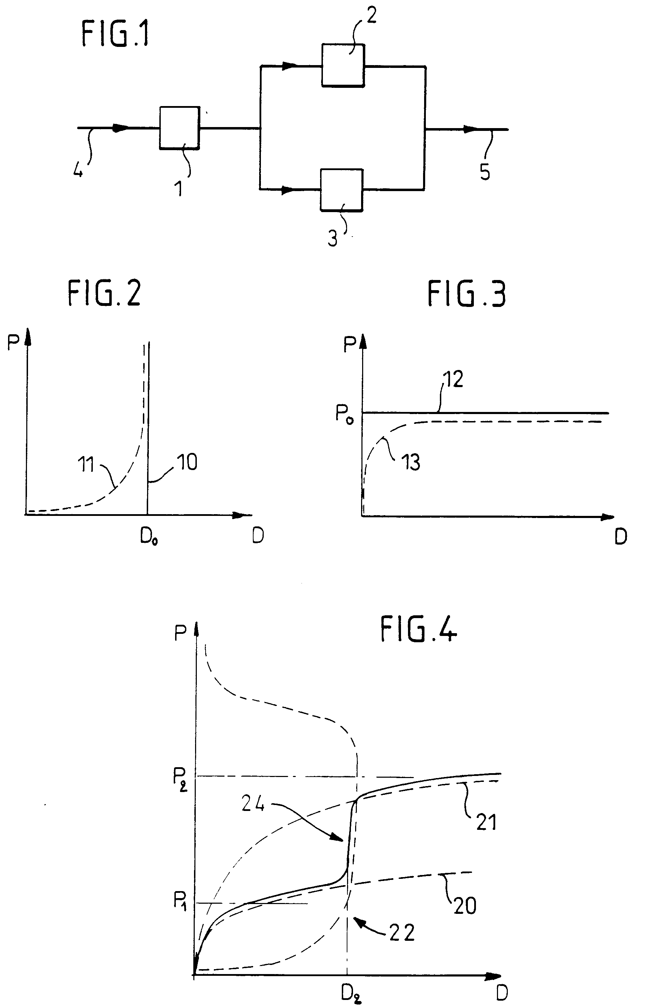

La figure 1 représente une première valve de régulation de pression 1 connectée en série avec l'ensemble constitué par une valve de régulation de débit 2 en parallèle avec une deuxième valve de régulation de pression 3. La valve 1 est située en amont de l'ensemble 2,3 de sorte que, lorsque ce dispositif a été chirurgicalement implanté, le cathéter amont 4 d'amenée de fluide céphalo-rachidien à l'entrée de la valve 1 a son extrémité libre au niveau des ventricules cérébraux, alors que le cathéter aval 5 réunissant les sorties des valves 2 et 3 a son extrémité libre dans la zone de drainage, par exemple dans la région lombaire.

La figure 2 représente la courbe caractéristique d'une valve telle que la valve 2. La courbe idéale 10 en trait plein montre qu'en théorie la valve 2 laisse passer un débit de fluide égal à D₀ quelque soit la pression différentielle entre son amont et son aval. En pratique, la courbe aura une allure telle que celle représentée en 11 en traits interrompus.

La figure 3 représente la courbe caractéristique d'une valve telle que 1 ou 3. Idéalement, cette courbe est celle représentée en 12 en trait plein pour laquelle la pression différentielle entre l'amont et l'aval est constante et égale à P₀ quelque soit le débit. En pratique on obtient une courbe comme celle représentée en 13 en traits interrompus.

On voit sur la figure 4, en 20 la courbe caractéristique de la valve 1, en 21 la courbe caractéristique de l'ensemble constitué par les valves 1 et 3 (la courbe caractéristique de la valve 3 est par conséquent constituée par la différence entre les courbes 21 et 20) et en 22 la courbe caractéristique de la valve 2.

On constatera que la courbe 22 possède, contrairement à la courbe 11, une zone de fermeture 23 pour une pression P₂ correspondant sensiblement à la pression de régulation de l'ensemble des valves 1 et 3.

La courbe caractéristique de l'ensemble 1-3 est représentée en 24 en trait plein.

Lorsque la pression différentielle au travers de l'ensemble du dispositif atteint une valeur P₁ déterminée par la valve de régulation de pression 1, la pression se stabilise jusqu'à ce que le débit atteigne la valeur D₂ imposée par la valve de régulation de débit 2. Le débit reste constant pour une plage de pression compris entre P₁ et P₂, la pression étant limitée à la valeur P₂ par la valve de régulation de pression 3.

Ce dispositif permet par conséquent bien d'obtenir le but recherché, à savoir d'obtenir un débit sensiblement constant pour toute une gamme de pression , ce débit étant par conséquent notamment indépendant de la position du patient.

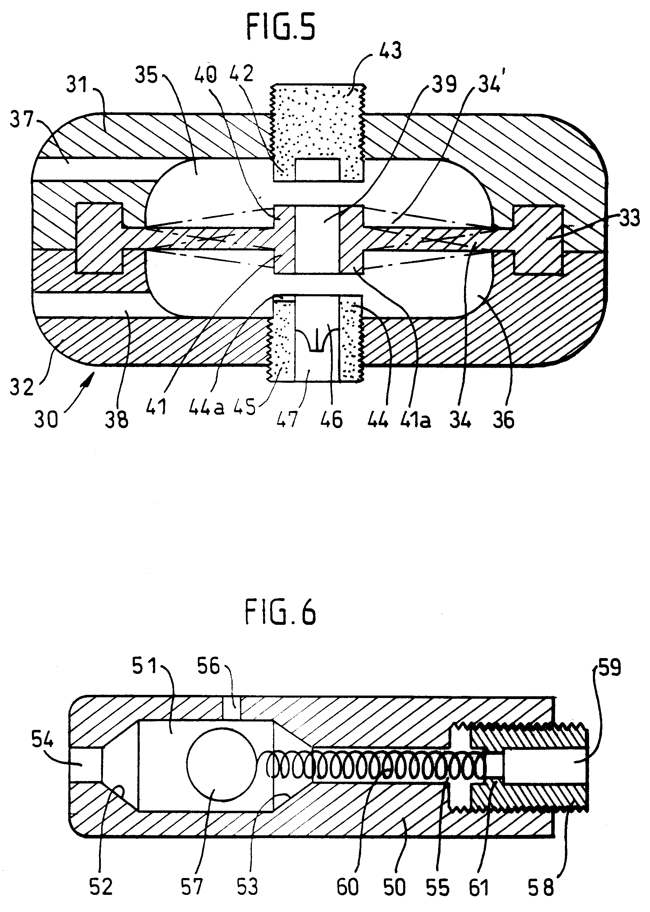

Si l'on se réfère maintenant à la figure 5, on voit un dispositif selon l'invention comprenant un boîtier 30 réalisé dans un matériau biocompatible et formé d'une partie supérieure 31 et d'une partie inférieure 32 toutes deux en forme ce coupelle et soudées entres elles le long de leurs bords périphériques de manière à former une cavité intérieure.

Les parties 31 et 32 possédent des rainures en vis à vis dans lesquelles sont maintenues le bourrelet périphérique 33 d'une membrane 34 séparant la cavité intérieure en une chambre amont 35 et une chambre aval 36.

Un conduit 37 traversant la paroi de la partie 31 est relié par tous moyens convenables au cathéter 4 pour former l'entrée du dispositif tandis qu'un conduit 38 formé dans la paroi de la partie 32 constitue une des sorties du dispositif reliée au cathéter 5.

La membrane 34 possède en son centre un orifice 39 faisant communiquer les chambres amont 35 et aval 36 dans la position médiane de la membrane représentée en trait plein au dessin, cet orifice 39 étant bordé du côté de la chambre amont 35 par un joint d'étanchéité 40 et du côté de la chambre aval 36 par un joint d'étanchéité 41. Les joints d'étanchéités 40 et 41 sont réalisés d'une seule pièce avec la membrane 34 et font saillies chacun d'un des côtés de cette membrane.

Un siège annulaire 42 est formé à l'extrémité axiale intérieure d'une vis 43 traversant la paroi supérieure de la partie 31 du boîtier coaxialement à l'ouverture 39, le siège 42 étant par conséquent apte à coopérer avec le joint 40.

De façon similaire, un siège 44 est formé à l'extrémité axiale intérieure d'une vis 45 traversant la paroi inférieure de la partie 32 du boîtier coaxialement à l'orifice 39 et a la vis 43, de sorte que le siège 44 peut coopérer avec le joint d'étanchéité 41 de la membrane.

La surface du siège 44 comporte ici des rainures radiales 44a autorisant une légère fuite lorsque le joint 41 est appliqué sur le siège 44. En variante, la surface d'étanchéité 41a du joint 41 pourrait présenter une forme tronconique s'évasant en direction du siège 44 de manière à former une lèvre souple permettant également une légère fuite.

Une valve à fente 46 (ou en "bec de canard") est disposée dans un conduit 47 formé axialement dans la vis 45.

La membrane 34, par exemple une membrane à soufflet, est prévue pour, au repos, être dans sa position représentée en trait mixte en 34' dans laquelle le joint 40 est en appui sur le siège 42. Dans ce cas, tout écoulement est par conséquent bloqué entre l'amont et l'aval du dispositif.

Lorsque la pression différentielle de part et d'autre de la membrane dépasse un certain seuil, le joint 40 se sépare du siège 42 permettant ainsi l'écoulement du fluide de l'amont vers l'aval. La membrane 34 et le siège 42 se comportent par conséquent comme la valve de régulation de pression 1 de la figure 1.

Lorsque la pression différentielle augmente, la membrane se déforme de plus en plus, le joint 41 se rapprochant par conséquent progressivement du siège 44. Plus la pression est forte et plus le joint 41 se rapproche du siège 44, formant ainsi une restriction annulaire qui diminue au fur et à mesure que la pression augmente. L'ensemble constitue par la membrane 34 et le siège 44 se comporte par conséquent comme la valve de régulation de débit 2.

Lorsque la pression différentielle atteint un certain seuil, le joint 41 vient s'appliquer sur le siège 44, isolant de nouveau les chambres amont 35 et aval 36 du dispositif tandis que la valve à fente 46 s'ouvre, déterminant ainsi la pression différentielle maximale entre l'amont et l'aval du dispositif.

Bien entendu, la sortie du conduit 47 est relié par tous moyens convenables au cathéter 5.

Si l'on se réfère maintenant à la figure 6, on voit un ensemble de valves contenues dans un boîtier 50 formant une cavité cylindrique 51 terminée à ces deux extrémités par les sièges côniques 52 et 53.

Un conduit 54 débouche dans l'axe du siège cônique 52 et un conduit 55 débouche dans l'axe du siège cônique 53.

Un orifice 56 est percé dans la paroi du boîtier 50 pour faire correspondre l'extérieur du boîtier à l'intérieur de la cavité 51.

Une bille 57 est disposée dans la cavité 51 pour se déplacer entre les sièges côniques 52 et 53.

Une vis 58 est vissée dans le boîtier 50 coaxialement à la cavité 51 et aux conduits 54 et 55, cette vis comportant un conduit axial 59 permettant de faire communiquer le conduit 55 avec l'extérieur du boîtier.

Un ressort 60 est placé dans le conduit 55 en appui d'une part sur la bille 57 et d'autre part sur un épaulement 61 formé à l'intérieur du conduit 59.

Le ressort 60 repousse par conséquent la bille 57 vers le siège conique 52 avec une force réglable par le serrage de la vis 58.

L'orifice 56 est relié à l'entrée d'une valve de régulation de pression de tout type connu.

Le dispositif de la figure 6 est implanté avec le conduit 54 relié à l'extrémité aval du cathéter 4 et le conduit 59 relié a l'extrémité amont du cathéter 5, à laquelle est également reliée la sortie de la valve de régulation de pression précitée.

En l'absence de pression différentielle entre les conduits 54 et 59, la bille 57 est appliquée par le ressort contre le siège 52. Lorsque la pression différentielle atteint un certain seuil, la bille 57 s'éloigne du siège conique 52 et se comporte par conséquent comme la valve de régulation de pression 1.

Lorsque la pression différentielle augmente, la bille 57 se déplace vers le siège conique 53 jusqu'à proximité de ce dernier où elle se comporte comme la valve de régulation de débit 2. Arrivée à un certain seuil de pression différentielle, la bille 57 se plaque contre le siège conique 53, fermant ainsi le passage entre les conduits 54 et 59. La valve de régulation de pression connectée entre l'orifice 56 et l'extrémité amont du cathéter 5 a sa pression de seuil réglée à la même valeur, de sorte qu'elle se comporte alors comme la valve de régulation de pression 3.

Le dispositif de la figure 6, associé à une valve de régulation de pression convenablement étalonnée, permet par conséquent d'obtenir également la courbe caractéristique pression/débit souhaitée.

## Revendications

1. Dispositif de drainage implantable pour le traitement de l'hydrocéphalie, comprenant une première valve de régulation de pression (1; 40,42; 52,57) en série avec une valve de régulation de débit (2; 41,44; 53,57), caractérisé par le fait qu'il comprend une deuxième valve de régulation de pression (3; 46) en parallèle avec ladite valve de régulation de débit, la deuxième valve de régulation de pression ayant une pression différentielle d'ouverture supérieure à celle de la première.

2. Dispositif selon la revendication 1, dans lequel la première valve de régulation de pression est disposée en série avec l'ensemble constitué par la valve de régulation de débit en parallèle avec la deuxième valve de régulation de pression.

3. Dispositif selon l'une quelconque des revendications 1 et 2, dans lequel ladite valve de régulation de débit comprend une membrane (34) délimitant avec un boitier (30), une chambre amont (35) et une chambre aval (36), ladite membrane de la valve de régulation de débit possédant une ouverture centrale (39) agencée pour faire communiquer les chambres amont et aval et délimitée par un joint annulaire (41) situé en vis à vis d'un siège (44) de forme correspondante formé dans le boîtier du côté de la chambre aval.

4. Dispositif selon la revendication 3, comportant des moyens permettant une fuite de la valve de régulation de débit dans son état fermé.

5. Dispositif selon l'une quelconque des revendications 1 à 4, dans lequel l'une au moins des valves de régulation de pression comprend une membrane (34) délimitant avec un boîtier (30), une chambre amont (35) et une chambre aval (36), ladite membrane de la valve de régulation de pression possédant une ouverture centrale (39) agencée pour faire communiquer les chambres amont et aval et délimitée par un joint annulaire (40) situé en vis à vis d'un siège (42) de forme correspondante formé dans le boîtier du côté de la chambre amont, le joint étant appliqué contre le siège pour une pression différentielle entre les chambres amont et aval inférieure à la pression de régulation de ladite valve.

6. Dispositif selon la revendication 5, dans lequel ladite valve de régulation de pression est la première valve de régulation de pression.

7. Dispositif selon l'ensemble des revendications 3 et 6, dans lequel une seule membrane est utilisée comme membrane de la valve de régulation de débit et comme membrane de la première valve de régulation de pression, un joint annulaire étant formé de chaque côté de la membrane autour de la même ouverture centrale et les deux sièges étant en conséquence formés en vis à vis dans le boîtier, l'une du côté de la chambre amont et l'autre du côté de la chambre aval.

8. Dispositif selon la revendication 3, dans lequel le conduit d'amenée à la deuxième valve de régulation de pression débouche dans ladite chambre aval à l'intérieur de la zône délimitée par le siège.

9. Dispositif selon l'une quelconque des revendications 3 à 8, dans lequel l'un au moins desdits sièges est formé dans une vis (43,45) coopérant avec un filetage formé dans la paroi du boîtier.

10. Dispositif selon l'ensemble des revendications 8 et 9, dans lequel la deuxième valve de régulation de pression est une valve à fente (46) disposée dans un perçage (47) traversant axialement la vis (45) supportant le siège de la valve de régulation de débit.

11. Dispositif selon la revendication 2, dans lequel la première valve de régulation de pression et la valve de régulation de débit sont formées d'une chambre cylindrique (51) dans laquelle une bille (57) est agencée pour se déplacer entre deux sièges coniques (52,53) d'extrémité, le conduit d'entrée (54) à ladite première valve de régulation de pression débouchant au centre du premier (52) de ces sièges coniques et le conduit de sortie (60) de la valve de régulation de débit débouchant au centre du deuxième (53) de ces sièges coniques, un ressort de tarage (60) poussant ladite bille en direction du premier siège conique, et le conduit d'amenée (56) à la deuxième valve de régulation de pression débouchant dans ladite chambre cylindrique.
